# EUROPEAN PATENT SPECIFICATION

(11) **EP 3 574 912 B1**
(45) Date of publication and mention of the grant of the patent: **17.02.2021**
(21) Application number: 18184987.8
(22) Date of filing: 23.07.2018
(51) Int. Cl.: A61K 36/8962, A61K 35/64, A61K 36/185, A61K 36/70, A61K 35/63, A61P 3/10

(54) **COMPOSITION FOR TREATING DIABETIC DISEASE**
ZUSAMMENSETZUNG ZUR BEHANDLUNG EINER DIABETISCHEN ERKRANKUNG
COMPOSITION POUR TRAITER UNE MALADIE DIABÉTIQUE

(30) Priority: 30.05.2018 KR 20180061788
(43) Date of publication of application: 04.12.2019
(73) Proprietor: Lee, Sam-Goo, Jeonju-si, Jeollabuk-do 54997 (KR)
(72) Inventor: Lee, Sam-Goo, Jeonju-si, Jeollabuk-do 54997 (KR)
(74) Representative: Cabinet Chaillot

(56) References cited:
- KR-A- 20160 096 307
- MI YOUNG AHN: "Anti-oxidative activity of db mice treated with glycosaminoglycan of cricket, Gryllus bimaculatus", THE FASEB JOURNAL, vol. 32, no. 1_supplement, 673.6, 1 April 2018 (2018-04-01), pages 1-1, XP002788311,
- JUAN ANTONIO GIMÉNEZ-BASTIDA ET AL: "Buckwheat as a Functional Food and Its Effects on Health", JOURNAL OF AGRICULTURAL AND FOOD CHEMISTRY, vol. 63, no. 36, 3 September 2015 (2015-09-03), pages 7896-7913, XP055546795, US ISSN: 0021-8561, DOI: 10.1021/acs.jafc.5b02498
- DONG-GI LEE ET AL: "Effect of rutin from tartary buckwheat sprout on serum glucose-lowering in animal model of type 2 diabetes", ACTA PHARMACEUTICA, vol. 66, no. 2, 1 June 2016 (2016-06-01), pages 297-302, XP055546790, DOI: 10.1515/acph-2016-0021
- MUHAMMAD SAJID HAMID AKASH ET AL: "Spice plant Allium cepa: Dietary supplement for treatment of type 2 diabetes mellitus", NUTRITION., vol. 30, no. 10, 1 October 2014 (2014-10-01), pages 1128-1137, XP055546803, US ISSN: 0899-9007, DOI: 10.1016/j.nut.2014.02.011
- Ogbonna J Ikechukwu ET AL: "The Antidiabetic Effects of The Bioactive Flavonoid (Kaempferol-3-O---D- 6{P-Coumaroyl} Glucopyranoside) Isolated From Allium cepa", Recent Patents on Anti-Infective Drug Discovery, 1 January 2016 (2016-01-01), pages 44-52, XP055546802, Retrieved from the Internet: URL:http://www.eurekaselect.com/136676/art icle
- KAREN M. STRAT ET AL: "Mechanisms by which cocoa flavanols improve metabolic syndrome and related disorders", THE JOURNAL OF NUTRITIONAL BIOCHEMISTRY, vol. 35, 1 September 2016 (2016-09-01), pages 1-21, XP055546813, AMSTERDAM, NL ISSN: 0955-2863, DOI: 10.1016/j.jnutbio.2015.12.008

## Description

### BACKGROUND OF THE INVENTION

### 1. Field of the Invention

The present invention relates to a composition for treating a diabetic disease, capable of remarkably improving blood glucose in Type 1 diabetes, which is insulin-dependent diabetes mellitus, as well as Type 2 diabetes which is non-insulin-dependent diabetes.

More particularly, the present invention relates to a composition for treating type I diabetic disease, capable of remarkably improving blood glucose within the short period of 4 weeks as a user has a nutritious meal substitution in one meal per day without the administration of insulin.

### 2. Description of the Related Art

Diabetes mellitus is a type of metabolic disease that lacks insulin secretion in the pancreas or does not function normally. It is characterized by high blood glucose concentration. Diabetes mellitus is divided into Type 1 and Type 2 diabetes.

Type 1 diabetes, which is called 'childhood diabetes', is caused since insulin is not produced as beta cells in a pancreas are destroyed. This is because a congenital or acquired destruction of beta cells in the pancreas cause the production of necessary insulin. Type 1 diabetes is a serious disease that makes a patient live while relying on insulin injections and insulin pumps.

Type 2 diabetes is a disease caused because insulin produced from a beta cell in the pancreas is relatively insufficient or caused because a cell fails to effectively burn glucose due to insulin resistance even though a sufficient amount of insulin.

Among them, Type 2 diabetes is mainly caused due to environment factors, such as high calorie, high fat, and high protein diet resulting from the westernization of eating habits, drinking, the lack of exercise, or stress. In addition, it is well-known that Type 2 diabetes may be caused due to the defect of genes and may be caused due to pancreas surgery, infection, or medicine.

As a symptom of diabetes, when the blood glucose level is high, thirst occurs and a lot of water is consumed, the urine volume increases. Accordingly, a patient frequently goes a bathroom, and the weight of the patient is lost. If the high blood glucose level is kept for 10- 20 years since diabetes occurred, various complications are incurred in the body of the patient. Representatively, the danger of retinopathy, renal dysfunction, neuropathy, cardiac dysfunction, stroke, peripheral nervous system disorders, or cardiovascular disease is significantly increased. Nevertheless there is present such a serious physical danger, only attempts to administrating insulin have been made to treat a diabetic patent, especially, a patent having Type 1 diabetes without fundamental treatment. Until now, there is no way other than the administration of insulin in modern medicine. Accordingly, the researches or studies have to be rapidly performed on the regeneration or the survival of a beta cell in a pancreas of a patient having diabetes regarded as a difficult problem in the modern medical world.

Although various medicines for diabetic patients are being studied and supplied, the patients are merely lowering diabetic levels by ingesting medicines rather than treating diabetes.

There are also a variety of folk remedies.

In other words, it is said that most of the foods that we eat are good for diabetes. As an example, mugwort is good for diabetes. However, doctors say that they should be careful about the intake of mugwort since even mugwort contains a lot of sugar.

It is known that an elm shell has a lot of saponin and lowers blood glucose and blood lipid, so the elm shell is helpful for controlling diabetes. In addition, it is well known that beet is good food for diabetes. The beet is called a red radish due to the unique red color thereof. Especially since onions are known to be good for diabetes, people drink onion juice every day. In addition, a research result has been reported in which buckwheat, pork potatoes, and red ginseng are good for diabetes. In addition, many food materials are known as folk remedies which are good for diabetes. However, when a large amount of food materials that are said to be good for such diabetes are ingested continuously, not only may side effects occur, but the continuous ingestion is difficult. In particular, since different results are caused depending on physical constitutions of patients, it is difficult to make a generalization that the above-described food materials are effective against diabetes. In general, diabetic patients have control the diabetic levels while medicines supplied by the hospital and performing a dietary therapy. Accordingly, the development of a new effective therapeutic agent having no side effect is strongly required.

Accordingly, the present inventor had found a material to improve diabetes and was astonished by the fact that Type 1 diabetes as well as Type 2 diabetes could be remarkably improved without administering insulin. In other words, it is found that the composition for treating diabetic disease according to the present invention is effective in regenerating or surviving or recovering beta cells in the pancreas of an animal having Type 1 diabetes, which are streptozotocin-induced, and in improving an amount of insulin secretion to 60-70% or more, only after four weeks from the ingestion of the composition.

Cited reference 1 suggests to a composition including a glucokinase activator for treatment of diabetes. Cited reference 1 provides a chemical composition including a glucokinase activator and one or more anti-diabetic agents.

Cited reference 2 suggests peptides for the treatment of Type 2 diabetes and complications of the Type 2 diabetes, and provides exenatide analogs for treating and preventing complications of Type 2 diabetes, such as diabetic neuropathy, muscular dystrophy, and corneal endothelium.

Cited reference 3 suggests an SGLT2 inhibitor for treating Type 1 diabetes, Type 2 diabetes, impaired glucose tolerance, or hyperglycemia and discloses a pharmaceutical composition including an SGLT2-inhibitor.

Cited reference 1 to cited reference 3 provide chemical therapeutic agents. As described above, the chemical therapeutic agents seriously cause side effects and have little advantages. Accordingly, the effectiveness of the chemical therapeutic agents may be degraded.

### List of Prior arts

### [Patent Documents]

Cited reference 1: Korean Unexamined Patent Publication No. 10-2015-0013838.
Cited reference 2: Korean Unexamined Patent Publication No. 10-2017-0041914.
Cited reference 3: Korean Unexamined Patent Publication No. 10-2011-0118668.
Cited reference 4: Korean Patent Registration No. 10-1200419.

### SUMMARY OF THE INVENTION

An object of the present invention is to provide a composition for treating a diabetic disease, capable of remarkably improving Type 1 diabetes and Type 2 diabetes while being used as a meal substitution.

According to the present invention, diabetic disease may be improved through a vegetable powder mixture comprising onion powders, buckwheat powders, and cacao nibs powders, and/or cricket powders.

Preferably, according the present invention, a vegetable powder mixture is prepared by mixing buckwheat powders, onion powders, and cacao nibs powders at the weight ratio of 50:25:25.

According to an embodiment of the present invention, the vegetable powder mixture is prepared by mixing 30-50% of buckwheat powders, 25-40% of onion powders, and 10-25% of cacao nibs powders.

Preferably, according to present invention, the cricket powders are mixed with the vegetable powder mixture at the weight ratio of 1:1.

Further, the diabetic disease in the claimed invention refers to Type 1 diabetes

As described above, according to the present invention, the diabetic disease may be significantly improved through a vegetable powder mixture containing buckwheat powders, onion powders, and cacao nibs powders, and/or cricket powders. Further, the effect of regenerating beta cells in the pancreas having the disorder of insulin secretion may be exhibited.

In addition, according to the present invention, a user may not only experience the improvement in blood glucose, but ingest higher-quality protein containing amino acid through the cricket powders. In addition, the user may ingest minerals such as magnesium and zinc, which exhibit anti-diabetic effects, and omega-3 unsaturated fatty acid and polyphenol. Further, as the user ingests the vegetable powder mixture, the user may be supplied with carbohydrate, and various vitamins and minerals.

### BRIEF DESCRIPTION OF THE DRAWINGS

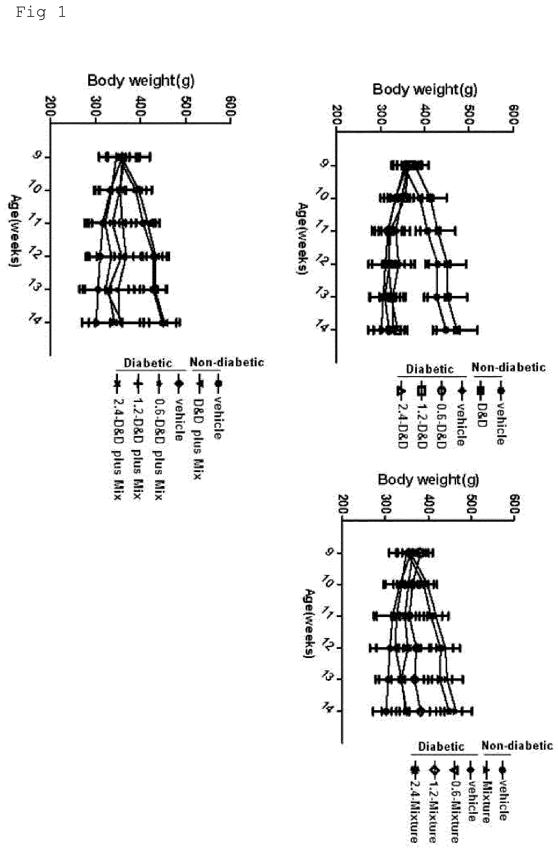

FIG. 1 illustrates graphs of the variation in the weights of experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

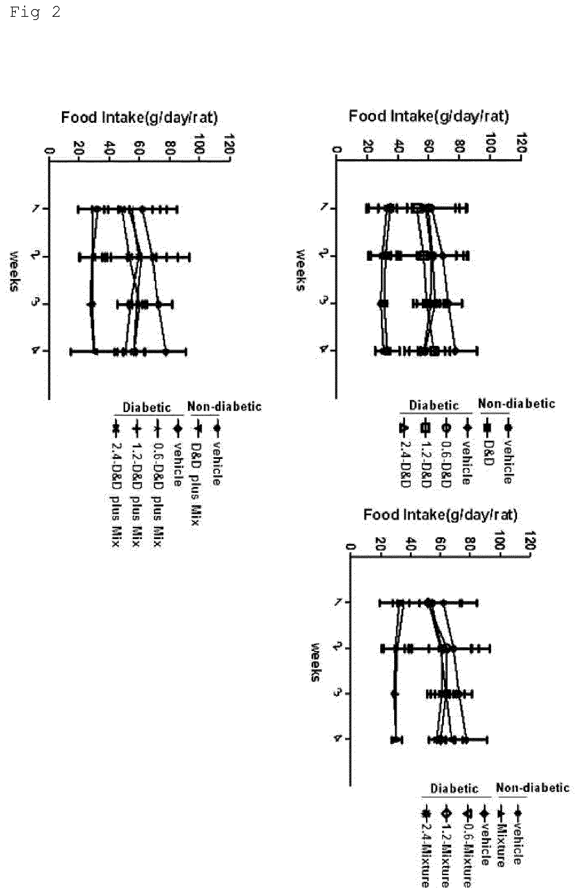

FIG. 2 illustrates graphs of the variation in the food intake of experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

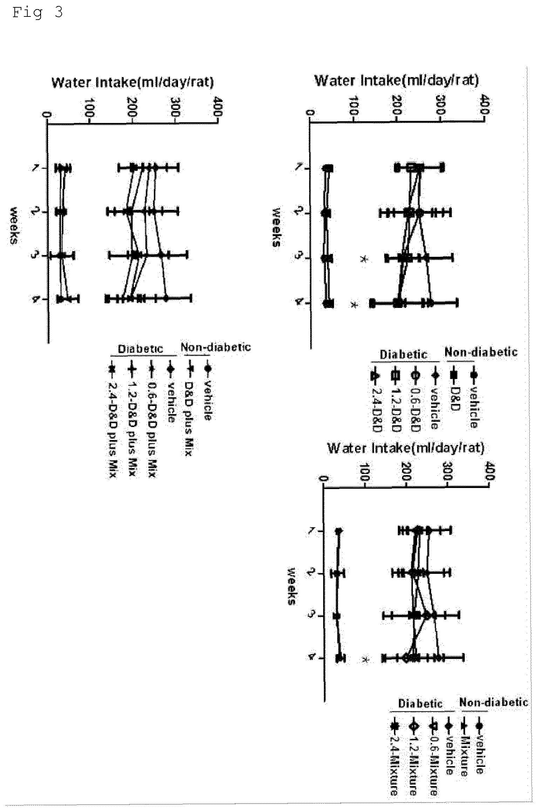

FIG. 3 illustrates graphs of the variation in the water intake of experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

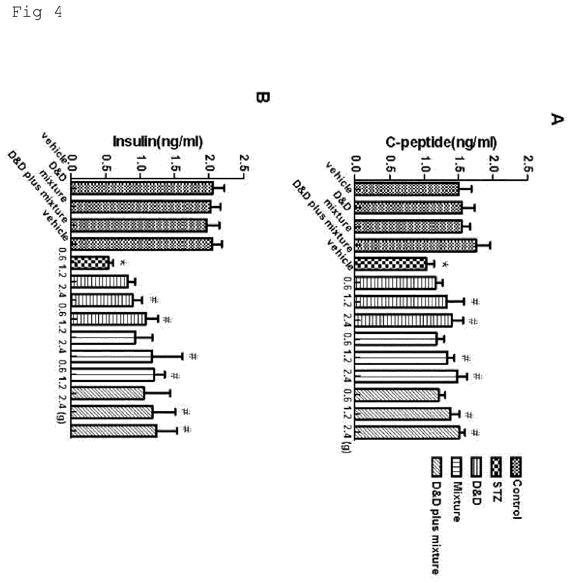

FIG. 4 illustrates graphs showing the numeric values obtained by measuring C-peptide and insulin using ELISA kits for experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

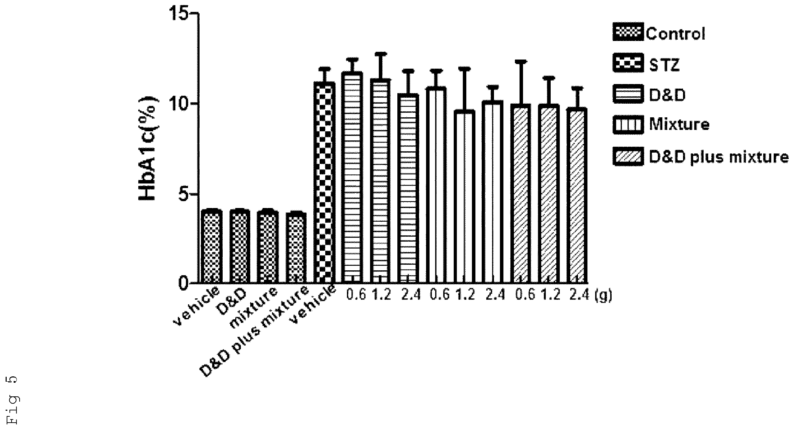

FIG. 5 illustrates graphs showing the measurement results of the variation in glycated hemoglobin values of experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

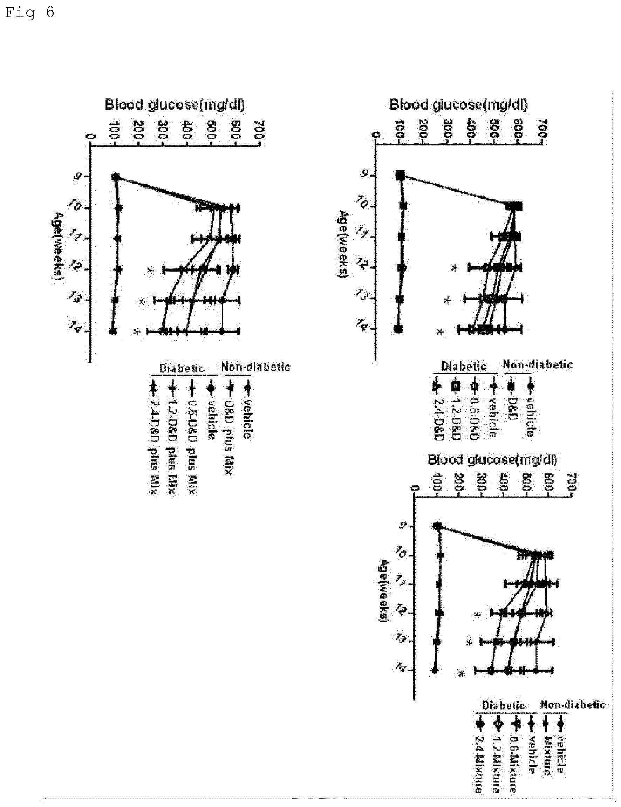

FIG. 6 illustrates graphs of showing the measurement results of the variation in fasting blood glucose of experimental rats induced with Type 1 diabetes after D&D, a vegetable powder mixture, and the D&D + the vegetable powder mixture are administrated to the experimental rats at a low concentration (0.6), an intermediate concentration (1.2), and a high concentration (2.4) according to the present invention.

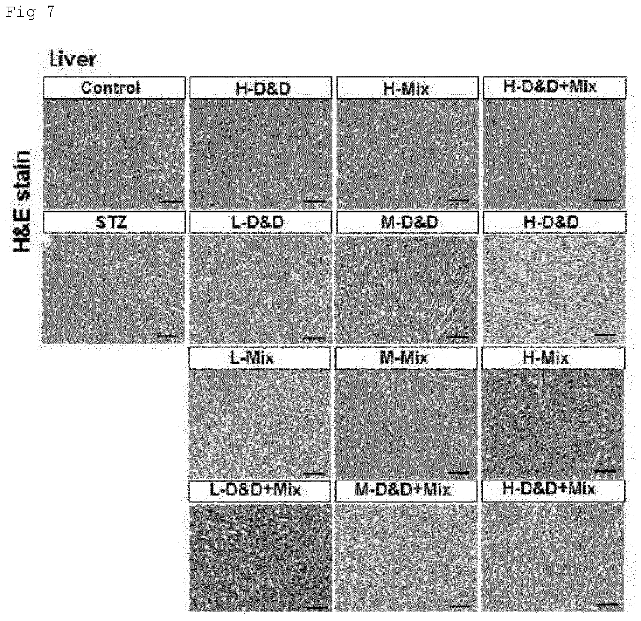

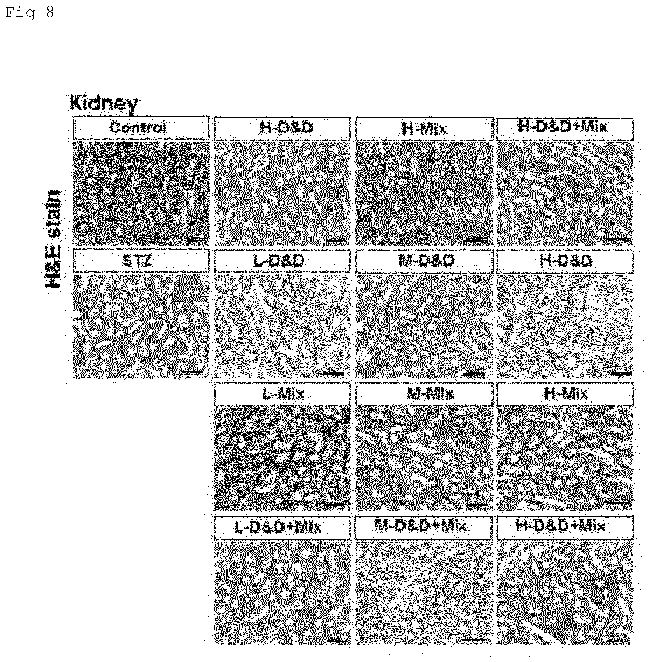

FIGS. 7 and 8 are views illustrating cell analysis results confirming influences exerted on internal organs of experimental rats induced with Type 1 diabetes after D&D, a vegetable powder mixture, and the D&D + the vegetable powder mixture are administrated to the experimental rats at a low concentration (0.6; L), an intermediate concentration (1.2; M), and a high concentration (2.4; H) according to the present invention.

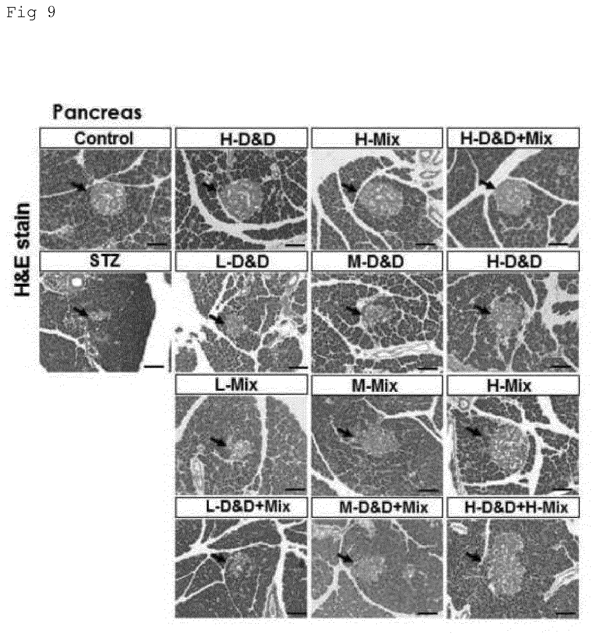

FIG. 9 illustrates views of pancreatic beta cells stained with Hematoxylin/Eosin to determine the functions of the pancreatic beta cells of experimental rats induced with Type 1 diabetes after D&D, a vegetable powder mixture, and the D&D + the vegetable powder mixture are administrated to the experimental rats at a low concentration (0.6; L), an intermediate concentration (1.2; M), and a high concentration (2.4; H) according to the present invention.

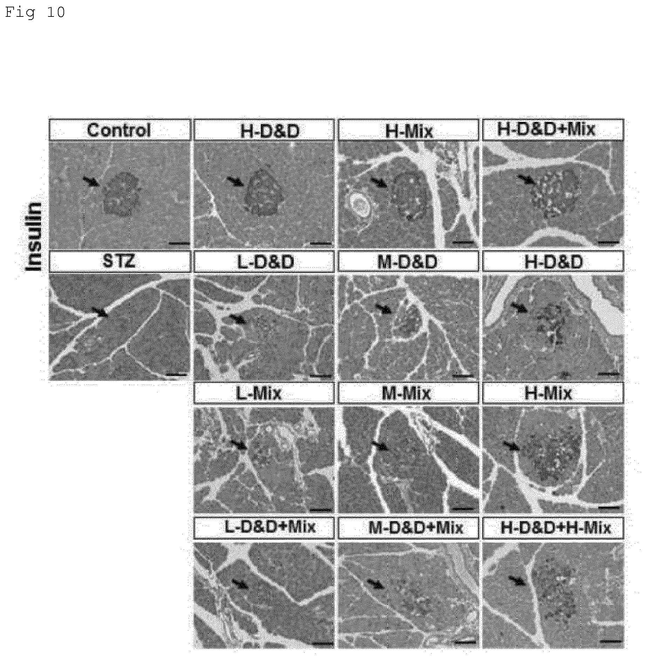

FIG. 10 illustrates views used to evaluate the expression degree of insulin in the stained cell of FIG. 9 through immunostaining using an insulin-specific antibody.

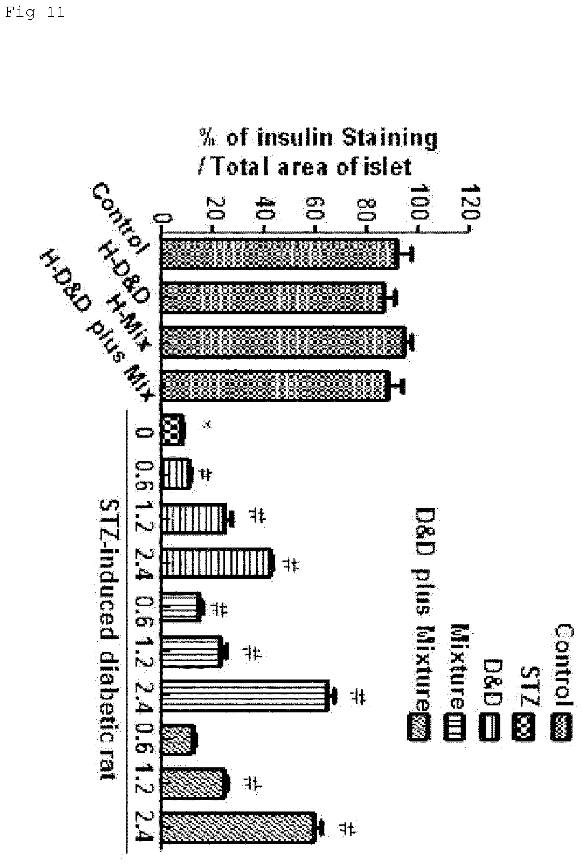

FIG. 11 is a graph illustrating the measurement result of the variation in the area of Islets in pancreases of experimental rats induced with Type 1 diabetes after D&D, a vegetable powder mixture, and the D&D + the vegetable powder mixture are administrated to the experimental rats at a low concentration (0.6; L), an intermediate concentration (1.2; M), and a high concentration (2.4; H) according to the present invention.

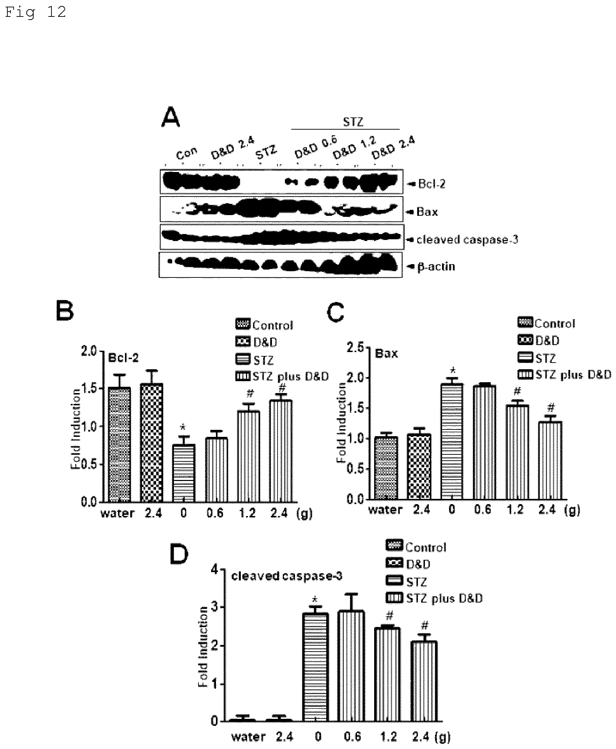

FIG. 12 illustrates graphs of the measurement results of the variation in Bcl-2 (Antiapoptotic), Bax (Proapoptotic), and Cleaved caspase-3 of a control group and groups administrated with D&D, STZ, and STZ + D&D depending on the concentrations.

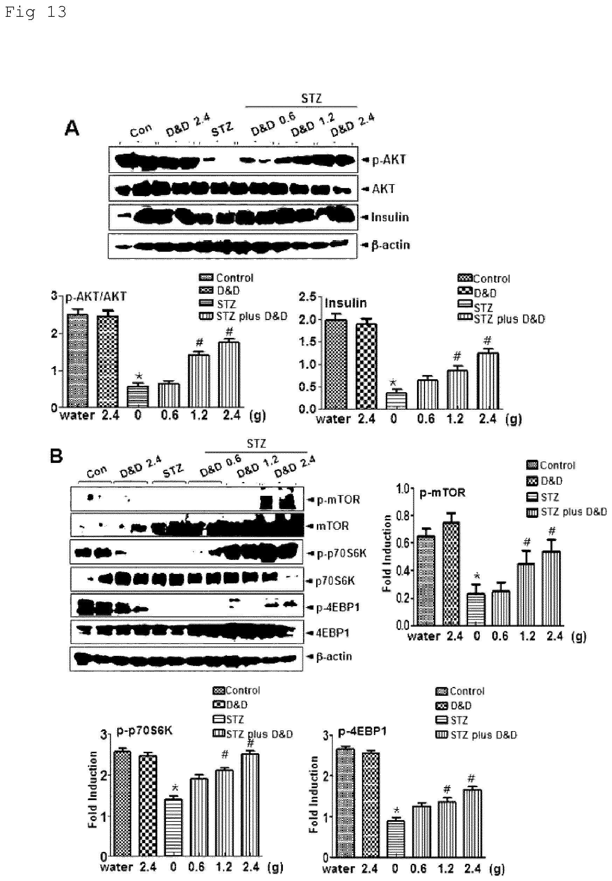

FIG. 13 illustrates graphs of the measurement result of the AKT/mTOR mechanism by western-blotting in the control group and the groups administrated with D&D, STZ, and STZ + D&D depending on the concentrations.

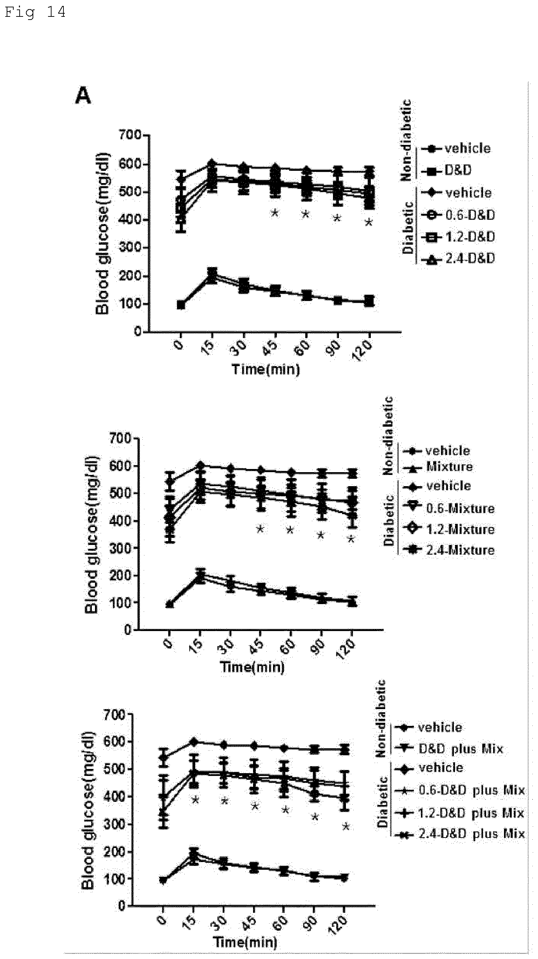

FIG. 14 illustrates graphs of the measurement results for the change in an IP glucose tolerance test (IPGTT) for experimental groups including experimental rats induced with Type 1 diabetes after D&D, a vegetable powder mixture, and the D&D + the vegetable powder mixture are administrated to the experimental rats at a low concentration (0.6; L), an intermediate concentration (1.2; M), and a high concentration (2.4; H) according to the present invention.

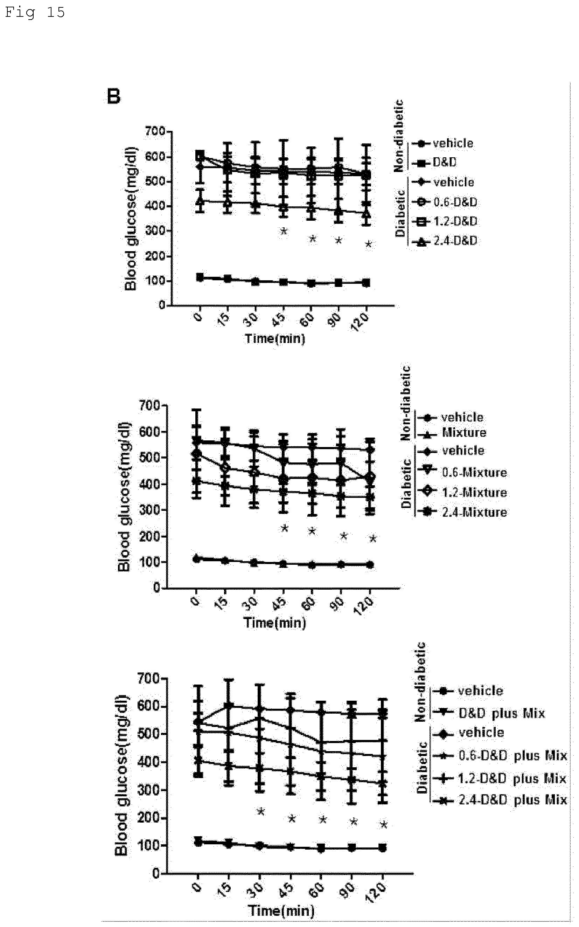

FIG. 15 illustrates graphs of the measurement results of improved insulin tolerance based on the change in an insulin tolerance test (ITT) for experimental groups including experimental rats induced with Type 1 diabetes after D&D, a vegetable powder mixture, and the D&D + the vegetable powder mixture are administrated to the experimental rats at a low concentration (0.6; L), an intermediate concentration (1.2; M), and a high concentration (2.4; H) according to the present invention.

### DETAILED DESCRIPTION OF THE INVENTION

Hereinafter, other objects and features will become apparent from the following description of embodiments with reference to the following figures.

In addition, unless otherwise defined, all terms used herein, including technical or scientific terms, have the same meanings as those generally understood by those skilled in the art to which the present invention pertains.

Such terms as those defined in a generally used dictionary are to be interpreted as having meanings equal to the contextual meanings in the relevant field of art, and are not to be interpreted as having ideal or excessively formal meanings unless clearly defined as having such in the present application.

### <Embodiment 1> Preparation of Cricket Powders

A cricket species used in the present invention is Gryllus bimaculatus. The cricket, Gryllus bimaculatus, was subject to hot-air drying or freezing-drying and was pulverized (in 100-300 mesh size) through a mill (a ball mill or a hammer mill). It is preferred that the freezing-drying is performed since the freezing-drying prevents nutrients, such as vitamins, from being destroyed. Cricket powders used in the present experiment were prepared by 239 BIO Ltd., and are, hereinafter, referred to as "D&D" (which is the brand name of the cricket powders).

### <Embodiment 2> Preparation of Vegetable powder mixture

A vegetable powder mixture was prepared by mixing buckwheat powders, onion powders, and cacao nibs powders at the weight ratio of 50: 25: 25. It is preferred that the vegetable powder mixture is supplied in the form of powders subject to freezing-drying and pulverizing since the form of powders prevent nutrients, such as vitamins, from being destroyed.

Buckwheat has a higher protein content and is rich in vitamins B1 and B2, nicotinic acid, and minerals, so the buckwheat is treated as a healthy diet. Especially, the buckwheat has the higher content of rutin which is effective in suppressing blood pressure, so the buckwheat has been used for the dietary therapy of patients with hypertension

Nutrients of 100 g of buckwheat contain 71.50 g of carbohydrate, 13.25 g of protein, 3.40 g of total fat, 10 g of dietary fiber, and cholesterol is not included at all. Among the nutrients contained in the buckwheat, vitamins include 30 µg of folic acid, 7.020 mg of niacin, 1.233 mg of pantothenic acid, 0.425 mg of riboflavin, and 0.101 mg of thiamine; electrolytes include 1 mg of sodium and 460 mg of potassium; minerals include 18 mg of calcium, 1.100 mg of copper, 2.20 mg of iron, 231 mg of magnesium, 1.300 mg of manganese, 374 mg of phosphorus, 8.3 µg of selenium, 2.40 mg of zinc, and amino acid includes 627 mg of lysine, 172 mg of methionine, and 192 mg of tryptophan.

In addition, the buckwheat contains corin, which is a nutrient belonging to the vitamin B group, to prevent fat from being accumulated in the liver. The corin serves as a source material of acetylcholine which decomposes alcohol in a human body. Accordingly, the buckwheat is helpful in neutralizing alcohol and relieving a hangover. The vegetable flavonoid component of the buckwheat is helpful in regenerating cells. The buckwheat is effective in curing a liver damaged due to alcohol or stress. In addition, it is known in oriental medicine that the buckwheat, which is classified as having a cold nature, is effective in lowering the heat of the human body and decreasing inflammation and produces an anti-aging effect resulting from antioxidant activity.

Since the buckwheat is abundant in dietary fiber, the buckwheat has an effect of preventing constipation. In addition, since the buckwheat is combined with a toxin, a toxic substance, and a carcinogen present in the internal organs of a human body, and is discharged to the outside of the body, the buckwheat protects a colonic mucosa. Furthermore, the buckwheat has the effect of lowering blood glucose and activating the function of the pancreas. This is because the buckwheat has abundant dietary fiber and rutin, which slows the absorption of glucose and regulates the change in blood glucose. In addition, since the buckwheat discharges active oxygen, the buckwheat prevents the damage to cells. Further, since the buckwheat produces diuretic action to allow a user to facilitate feces, and strengthens capillary blood vessels. Accordingly, the buckwheat is effective in preventing adult diseases. The buckwheat has abundant selenium to prevent blood in a blood vessel from being clotted, which is effected in cardiovascular diseases such as myocardial infarction, angina pectoris, and a cerebral stroke.

An onion contains an ingredient of propyl disulphide. The disulfide is effective in lowering the viscosity of the blood and clearing the blood by dissolving unnecessary fat and cholesterol in the body and thus in treating hyperlipidemia. In addition, the propyl disulphide is effective in preventing the blood from being clotted and decomposing the blood clot. Accordingly, the propyl disulphide has an effect of preventing atherosclerosis and hyperlipidemia and of lowering blood pressure. In addition, the propyl disulphide promotes metabolism, and prevents body fat from being accumulated to prevent obesity in advance, thereby preventing diabetes resulting from obesity. The propyl disulphide is rich in a raw onion. Accordingly, in the present invention, onion, which is not heated, is preferably used.

In addition, the onion has a large amount of chrome. The chrome is a micro-mineral to maintain homeostasis of glucose metabolism. Since the chrome promotes insulin action, the chrome is helpful in adjusting blood glucose.

The cacao nibs is obtained by fermenting cacao beans obtained from a cacao fruit, drying and roasting the fermented cacao beans, peeling the cacao beans, and crushing the cacao beans in small size. The cacao nibs contain a large amount of fiber to help digestion and improve blood sugar and cholesterol. The cacao nibs contain magnesium and iron powders. The cacao nibs abundantly have antioxidant ingredients to cope with cell mutant caused by oxygen stress, to cope with aging, to prevent inflammation, and to increase immunity. Accordingly, the cacao nibs may lower blood pressure. In addition, since the cacao nibs are rich in stearic acid and oleic acid, the cacao nibs effectively improve blood cholesterol.

Actually, the cacao nibs have been spotlighted around the world as it is known that the incidence of heart disease of Indians eating the cacao nibs has been lowered.

Meanwhile, since the buckwheat is classified as having a cold nature in oriental medicine, it is not recommended for a user having slow metabolism to ingest a large amount of buckwheat. In other words, it is known that it is better to reduce the intake of buckwheat for a person who suffers a stomachache and looses bowels when eating cold food. When a user intakes a large amount of buckwheat every day, the user has a problem with digestion and a severe trouble in bowels.

Therefore, according to the present invention, the proportion of the buckwheat may be lowered to 30 % based on the physical constitution of a patient to be administered into the patient.

Meanwhile, although the blood glucose may be controlled by ingesting only onion, it is difficult for the patient to continuously ingest the onion. In other words, the patient has to steadily ingest a large amount of onion every day so as to control the blood glucose by ingesting the onion. However, the patient may have a severe stomachache and a burn feeling in the stomach of the patient due to the ingestion of the onion. In addition, the patient may not easily ingest the onion due to the unique flavor and taste of the onion. Therefore, in the present invention, it is preferred to provide 25% of onion for administration. The onion has a warm nature which is opposite to that of the buckwheat. Accordingly, when the proportion of the buckwheat is lowered to 30% for administration, the proportion of the onion may be increased to 45% for administration. In this case, it is preferred that the mixture ratio of buckwheat and onion does not exceed 90%. It is more preferred that the mixture ratio of buckwheat and onion is within 75%.

If the mixture ratio of buckwheat and onion exceeds 90%, since any one of the cold nature of the buckwheat and the warm nature of the onion excessively predominates over the remaining nature, the vegetable powder mixture may not be applied to a person having slow metabolism or fast metabolism. In addition, if the mixture ratio of buckwheat and onion is less than 90%, the effect of preventing or treating diabetes may be slightly produced.

In addition, although the cacao nibs have effects of lowering triglycerides in blood and of improving blood flow, since the unique bitter tastes of the cacao nibs are strong, it is preferred that the proportion of the cacao nibs does not exceed the maximum proportion of 25%.

Preferably, the vegetable powder mixture was prepared by mixing 30-50% of buckwheat powders, 25-40% of onion powders, and 10-25% of cacao nibs powders.

Meanwhile, according to the present invention, it is preferred that the vegetable powder mixture is prepared by pulverizing the above ingredients and then mixing the ingredients.

### <Embodiment 3>

Embodiment 3 employed the vegetable powder mixture obtained by mixing the cricket powders obtained through Embodiment 1 and the vegetable powder mixture prepared through Embodiment 2 at the weight ratio of 1:1.

It was observed that the D&D, the vegetable powder mixture, and the mixture (hereinafter, referred to as "the D&D + the vegetable powder mixture") of the D&D and the vegetable powder mixture, which were prepared through Embodiment 1 to Embodiment 3 are effective for type 1 diabetes. The following experiment was performed to verify the regeneration of pancreatic beta cells which are completely destroyed.

The following experiment was accredited with accreditation No. cuh-IACUC-170316-6 on Nov. 08, 2017 by Non-Clinical Evaluation Center of Biomedical Research Institute at Chonbuk National University, which is Association for Assessment and Accreditation of Laboratory Animal Care International (AAALAC International).

In the following experimental example, each of D&D, the vegetable powder mixture, and the D&D + the vegetable powder mixture was administrated into experiment rats in dose of 1.2 g two times per day. The administration manner was oral administration.

In addition, each of the D&D, the vegetable powder mixture, and the D&D + the vegetable powder mixture was administrated at a low concentration (L), a middle concentration (M), and a high concentration (H). Regarding the concentration, when 40 g/60 kg or more is a maximum based on a human, 6.5 g/kg is set to a base value in the case of the experimental rate, in which 6.5 g/kg is obtained by rounding a value under a decimal point of 6.45 g which is a division result of 40 g/60 kg by 6.2. The value of the intermediate concentration is set to 3.25 g/kg which is 1/2 of 6.25 g, and the value of the low concentration is set to 1.63 g/kg which is 1/2 of 3.25 g/kg.

When the above value is changed to a concentration to be orally administrated to the experimental rat, the low concentration, the intermediate concentration, and the high concentration are set to 0.6 g, 1.2 g, and 2.4 g, respectively, thereby determining an administration amount per day.

### <Experimental Example 1>: Formation of Diabetic disease model

Regarding the cricket powders prepared through Embodiment 1, after fasting a 9 week-old experimental animal (rat) for 12 hours, Streptozotocin (STZ, S0130, Sigma-Aldrich, USA), which specifically acts to a beta cell in the pancreas and does not affect other organs, was dissolved in 0.1 M citrate buffer (pH 4.5), and the result was made to have the concentration of 65 mg/kg and injected into the experimental animal through intraperitoneal (IP) injection such that diabetes were induced from the experimental animal.

Regarding the determination of diabetes induction, after seven days (10 weeks) from the injection of the STZ, blood collected from the tail vein of the experimental rat was measured by a blood glucose meter. In this case, the experimental rat having the blood glucose of 300 mg/dl was regarded as a diabetic rate. All experimental groups were classified into a normal group, a diabetic control group, and a diabetic test group and raised such that the experimental groups have average weights approximate to each other. The experimental groups were raised for four weeks (or 11 weeks to 14 weeks) while being administrated with the test samples of the D&D, the vegetable powder mixture, and the D&D + the vegetable powder mixture after the determination of diabetes induction. Simultaneously, the variation in the blood glucose of the experimental groups was examined by measuring the weight, the feed consumption, the quantity of water, the blood glucose, the glycated hemoglobin beta cells, and the insulin secretion at the interval of one week.

### <Experimental example 2> Measurement of Dietary Efficiency

The weight increment and the food intake were measured at a time interval of one week from the start date of experiment while a sufficient amount of fodders and water was being supplied to the experimental groups. The food intake was calculated by subtracting the remaining amount of fodders from an amount of supplied fodders. A food efficiency ratio (FER) was calculated by dividing a weight increment for an experimental period by a food intake for the period.

Regarding the variation in weight, after 7 days (or 10 weeks) of the experimental period as illustrated in FIG. 1, although the weights of the experimental rates in the normal group are continuously increased, the weights of the experimental rates in the diabetic control group was decreased. In FIG. 1, the term "vehicle" represents a group to which regular fodders are supplied.

FIG. 1 does not show the meaningful result except that the weights are more or less increased in the experimental rates induced with diabetes and in groups to which the D&D, the vegetable powder mixture, and the D&D + the vegetable powder mixture are supplied. This result is presumed to be shown for the following reasons. As the use of glucose by cells is lowered in a diabetic state and thus the metabolism features are shown as in a starving state, so the weight loss is shown in the present experiment However, in the case of the groups administrated with the D&D, the vegetable powder mixture, and the D&D + the vegetable powder mixture having high concentration, higher-content amino acid is administrated and introduced into skeletal muscle to increase protein synthesis so that a body weight is increased.

Meanwhile, a food intake per day on weekdays shown in FIG. 2 and a water intake per day on weekdays shown in FIG. 3 are increased in the diabetic control group as compared with the normal group and significantly decreased in groups administrated with the D&D, the vegetable powder mixture, and the D&D + the vegetable powder mixture having high concentration

### <Experimental Example 3> Biochemical Analysis Technology in Blood

### 1) C-peptide

### ① Analysis Overview

C-peptide is a substance produced in a procedure of decomposing proinsulin, which reflects the secretion of insulin.

### (2) Experimental Manner

After plasma was isolated from blood samples for measurement, the plasma was analyzed using a C-peptide ELISA kit. The measurement for the analysis was performed according to the manual of the kit.

### 2) HbA1c

### ① Analysis Overview

Glycated hemoglobin is a biomarker for measuring the change in blood glucose for a long term.

### ② Experimental Manner

For the measurement, glycated hemoglobin was analyzed through collected blood samples in a clinical laboratory of a hospital. The measurement for the analysis was performed according to the manual of the kit.

### 3) Insulin

### ① Experimental Manner

After plasma was isolated from blood samples for measurement, the plasma was analyzed using an Insulin ELISA kit.

First, the measurement results of an experimental rat using C-peptide and insulin ELISA kits are shown in FIG. 4.

C-peptide is produced at the same proportion as that of insulin in the procedure of decomposing proinsulin. However, the C-peptide exists without being used in a liver or another peripheral tissue, which is different from the insulin. In addition, the C-peptide has a half-life longer than that of the insulin and thus is easily measured. The measurement value of the C-peptide represents the secretion degree of the insulin.

Arterial blood and venous blood show mutually different fasting plasma insulin concentrations, and the fasting plasma insulin concentration is in the normal range of 13-39 mU/l in the case of the venous blood.

As illustrated in FIG. 4, it was confirmed that the concentration of C-peptide in blood was significantly increased in the groups administrated with the D&D, the vegetable powder mixture, and the D&D + the vegetable powder mixture having high concentration. In general, when STZ was administrated, beta cells in the pancreas were destroyed, the insufficient production of insulin caused metabolic disorder, and the concentration of blood glucose was increased. Therefore, the D&D, the vegetable powder mixture, and the D&D + the vegetable powder mixture having high concentration (2.4; H), which were used in the present experimental example, may recover the function of beta cells in the pancreas to promote the secretion of insulin.

Next, regarding glycated hemoglobin, HbA1c, which is a part of the hemoglobin in a red blood cell, is slowly bound to glucose non-enzymatically to be saccharified during the survival of the red blood cell. Accordingly, the degree of saccharification of HbA1c is used to estimate the concentration of blood glucose during the survival of the red blood cell.

Therefore, glycated hemoglobin has been widely used as the most basic index representing glucose control. The average concentration of blood glucose for the last three months was analyzed based on glycated hemoglobin values.

**Table 1**

| | HbA1c |
|---|---|
| Control group | 4.0 |
| D&D | 3.99 |
| Vegetable powder mixture | 3.93 |
| D&D + Vegetable powder mixture | 3.83 |
| Diabetic group -control group | 11.11 |
| Diabetic group -0.6D&D | 11.66 |
| Diabetic group -1.2D&D | 11.31 |
| Diabetic group -2.4D&D | 10.47 |
| Diabetic group -0.6Mix | 10.81 |
| Diabetic group -1.2Mix | 9.54 |
| Diabetic group -2.4Mix | 10.08 |
| Diabetic group -0.6+Mix | 9.87 |
| Diabetic group -1.2+Mix | 9.88 |
| Diabetic group -2.4+Mix | 9.65 |

As illustrated in table 1 and FIG. 5, it was confirmed that there is the tendency in which glycated hemoglobin is decreased in the groups administrated with the D&D, the vegetable powder mixture, and the D&D + the vegetable powder mixture having high concentration (2.4; H) when the glycated hemoglobin was analyzed. Especially, it is a significantly great result that glycated hemoglobin values are decreased by about 1-2 without the administration of medicine or insulin for four weeks which is a very short term. The lowest glycated hemoglobin value is obtained in the case of 1.2 g of vegetable powder mixture, which is obtained due to the error of some data when numerical data is checked.

### <Experimental Example 4> Measurement of Blood Glucose

The blood glucose was measured using a blood glucose meter by collecting trail vein blood in the same time every week after fasting an experimental rat for 12 hours while the experimental rat is allowed to freely drink.

The fasting plasma glucose was measured from venous blood after fasting the experimental rat for at least 8 hours and the concentration of the fasting plasma glucose was expressed in mmol/L or mg/dl.

The concentration of the normal fasting plasma glucose was enhanced to 6.1 mmol/L (110 mg/dl) or less, and the concentration of two-hour blood glucose after feeding was limited to 7.8 mmol/L (140 mg/dl).

**Table 2**

| | 9 weeks | 10 weeks | 11 weeks | 12 weeks | 13 weeks | 14 weeks |
|---|---|---|---|---|---|---|
| Control group | 107.38 | 118.75 | 110.25 | 115.50 | 103.00 | 91.88 |
| D&D | 105.63 | 117.13 | 110.00 | 108.63 | 101.50 | 97.75 |
| Diabetic group - control group | 102.90 | 584.20 | 588.40 | 592.00 | 547.10 | 545.00 |
| Diabetic group -0.6D&D | 105.43 | 590.14 | 577.86 | 531.86 | 510.86 | 478.00 |
| Diabetic group -1.2D&D | 103.88 | 591.88 | 567.00 | 519.63 | 491.88 | 452.14 |
| Diabetic group -2.4D&D | 103.20 | 585.00 | 537.80 | 475.80 | 454.80 | 411.10 |

**Table 3**

| | 9 weeks | 10 weeks | 11 weeks | 12 weeks | 13 weeks | 14 weeks |
|---|---|---|---|---|---|---|
| Control group | 107.38 | 118.75 | 110.25 | 115.50 | 103.00 | 91.88 |
| Vegetable powder mixture | 112.86 | 115.71 | 113.57 | 109.86 | 97.57 | 96.43 |
| Diabetic group - control group | 102.90 | 584.20 | 588.40 | 592.00 | 547.10 | 545.00 |
| Diabetic group - 0.6Mix | 101.13 | 552.13 | 548.50 | 482.50 | 442.43 | 419.29 |
| Diabetic group - 1.2Mix | 102.25 | 540.88 | 520.63 | 478.38 | 447.25 | 419.63 |
| Diabetic group - 2.4Mix | 98.50 | 538.5 0 | 492.1 0 | 397.60 | 370.80 | 367.20 |

**Table 4**

| | 9 weeks | 10 weeks | 11 weeks | 12 weeks | 13 weeks | 14 weeks |
|---|---|---|---|---|---|---|
| Control group | 107.38 | 118.75 | 110.25 | 115.50 | 103.00 | 91.88 |
| Plus Vegetable powder mixture | 106.00 | 115.00 | 112.14 | 111.71 | 103.14 | 94.86 |
| Diabetic group -control group | 102.90 | 584.20 | 588.40 | 592.00 | 547.10 | 545.00 |
| Diabetic group | 100.43 | 544.43 | 531.43 | 476.86 | 425.00 | 401.71 |
| -0.6+Mix | | | | | | |
| Diabetic group -1.2+Mix | 104.63 | 535.25 | 540.00 | 456.25 | 426.50 | 398.13 |
| Diabetic group -2.4+Mix | 104.56 | 516.00 | 495.22 | 412.22 | 371.00 | 350.11 |

The variation in fasting plasma glucose, as illustrated in FIG. 6 (see table 2 to table 4), was remarkably increased in the diabetic-control group as compared with the normal group and was significantly decreased in the groups administrated with the D&D, the vegetable powder mixture, and the D&D + the vegetable powder mixture having high concentration. This result teaches that the blood glucose of a diabetic model with hyperglycemia is improved as the D&D, the vegetable powder mixture, and the D&D + the vegetable powder mixture having high concentration are administrated into the diabetic model.

### <Experimental Example 5> Influence on Organ

A toxicity experiment was performed using the possibility of causing damage to the liver or kidney of an experimental rat as the D&D and/or the vegetable powder mixture was continuously administrated to the experimental rat. FIGS. 7 and 8 illustrate the results obtained by examining the influence exerted on the liver or kidney in the procedure of overcoming a diabetic disease according to the present invention. As illustrated in photographs of FIGS. 7 and 8, it was observed that the present invention does not cause the meaningful change in the liver or kidney. Accordingly, the present invention is not considered to produce an adverse effect on the liver or kidney.

### <Experimental Example 6> Functional Measurement of beta Cells in Pancreas

### 1) Pancreatic tissue staining

The pancreas of a white rat was extracted, fixed in 3.7 formalin, and then dehydrated using an automatic tissue processor. A paraffin block was prepared using paraffin embedding machine. The paraffin block was cut with a microtome to prepare a 4 µm slice. The prepared slice was de-paraffinized and dehydrated using xylene and alcohol and stained with hematoxylin & eosin (H&E). Then, the prepared slice was hydrated and then observed using an optical microscope.

Regarding measurement of insulin present in a beta cell of a pancreatic tissue, insulin serving as an index to measure the function of the beta cells was analyzed by immunohistochemistry to show relative comparison between experimental groups. According to a result of immunohistochemical staining of insulin of the organ, the expression of insulin in the pancreatic Islets beta cells of the type 1 diabetes model was reduced. In contrast, the groups administrated with the D&D, the vegetable powder mixture, and the D&D + the vegetable powder mixture having high concentration (2.4) expressed insulin in the pancreatic Islets beta cells which were remarkably increased by at least 60% in four weeks. This strongly teaches that insulin secretion was promoted as the function of the beta cell was recovered.

Insulin is the most important hormone in the control of blood glucose of a human body and is produced by a beta cell in the pancreas. It is well known that, when STZ is administrated to an experimental rat, a pancreatic beta cell of the experimental rat is destroyed, thereby making the pancreatic beta cell abnormal.

According to the present experiment, when inducing the increase in blood insulin concentration of the groups administrated with the D&D, the vegetable powder mixture, and the D&D + the vegetable powder mixture having high concentration, there was checked an effect of insulin secretion of a pancreatic beta cell in an individual group administrated with the D&D, the vegetable powder mixture, and the D&D + the vegetable powder mixture.

In other words, the D&D, the vegetable powder mixture, and the D&D + the vegetable powder mixture are administrated to Type 1 diabetes model, which having diabetes induced by STZ, for four weeks and then the slice of a pancreas was obtained and stained with hematoxylin/eosin to identify a beta cell (see FIG. 9). To identify insulin-producing beta cells in the pancreas, the expression degree of insulin was evaluated through tissue immunostaining using an insulin-specific antibody (see FIG. 10) .

The insulin-producing beta cell in a pancreas was markedly damaged in the diabetic control group. When the D&D, the vegetable powder mixture, and the D&D + the vegetable powder mixture having high concentration were administrated, the area of Islets in the pancreas was remarkably increased only in four weeks and the number of beta cells was significantly increased (see FIG. 11).

In particular, as positive beta cells were increased in the groups administrated with the D&D, the vegetable powder mixture, and the D&D + the vegetable powder mixture having high concentration, a concentration-dependent effect was verified. According to this experimental example, it is determined that the D&D, the vegetable powder mixture, and the D&D + the vegetable powder mixture are importantly involved in regenerating or recovering the beta cells, which is an important result for finding out the action mechanism of the effect of lowering blood glucose.

Hereinafter, description will be made on the mechanism that the D&D allows the beta cells to survive and regulates the functional recovery in associated with a diabetic model from the STZ. To prove the effect of the D&D for B-cell lymphoma 2 (BCL2)-type proteins in the pancreas, the expression of B-cell lymphoma 2 (BCL2)-type proteins and BCL-2 proteins was measured through western-blotting.

As illustrated in FIG. 12, the group administrated with the D & D was decreased in Bax expression and cleaved caspase-3, but increased in Bcl-2 expression. This is a result showing that the beta cell apoptosis is suppressed by the cricket powders.

Meanwhile, the effect of the cricket powders on AKT and mTOR expression in the pancreas of an experimental rat having Type 1 diabetes is produced as follows.

According to the previous studies, it has been reported that an experimental rat administrated with rapamycin, which inhibits a mammalian target of rapamycin complex 1 (mTORC1) activity, decrease beta cell mass and glucose tolerance. Therefore, in the present experimental example, an influence by the activation of mTOR was considered since the D&D was a material having a rich amino acid. The AKT/mTOR mechanism was demonstrated by Western blotting. According to the experimental result, it was recognized that the cricket powders increased phosphorylation and insulin expression of AKT as illustrated in FIG. 13. In addition, the cricket powders increased the phosphorylation of mTOR, p70S6K and 4EBP1 in a rat having diabetes induced by STZ.

### <Experimental Example 7> Measurement of IPGTT and ITT

To determine a glucose tolerance effect as the D&D, the vegetable powder mixture, and the D&D + the vegetable powder mixture were administrated to an experimental rat having diabetes, glucose tolerance and insulin resistance of the experimental rat were measured after four weeks of the experiment

In an IP Glucose Tolerance Test (IPGTT), the experimental rat was subject to 12-hour fasting in two days before the end of the experiment and subject to IP injection with 1g/kg of a glucose solution. Then, blood was collected from a rat tail vein after 15, 30, 45, 60, 90, and 120 mins from IP injection and blood glucose was measured. In an Insulin Tolerance Test (ITT), the experimental rat was subject to 5-hour fasting in the final week before the end of the experiment and subject to IP injection with 0.75 U/kg of an insulin solution (sigma-Aldrich, USA). Then, blood was collected from a rat tail vein after 0, 15, 30, 45, 60, 90, and 120 mins from IP injection and blood glucose was measured.

**Table 5**

| | 0 min | 15 mins | 30 mins | 45 mins | 60 mins | 90 mins | 120 mins |
|---|---|---|---|---|---|---|---|
| Control group | 94.125 | 193.125 | 159.125 | 143.375 | 130.125 | 113.250 | 102.750 |
| D&D | 96.625 | 207.125 | 175.500 | 147.125 | 129.000 | 113.125 | 110.625 |
| Diabetic group -control group | 544.000 | 601.000 | 590.400 | 585.200 | 577.900 | 573.700 | 573.400 |
| Diabetic group -0.6 D&D | 472.000 | 558.143 | 544.286 | 536.571 | 528.143 | 519.000 | 503.000 |
| Diabetic group -1.2 D&D | 444.000 | 546.571 | 537.143 | 529.714 | 518.286 | 507.286 | 493.571 |
| Diabetic group -2.4 D&D | 405.100 | 542.300 | 534.200 | 525.200 | 512.000 | 495.000 | 478.200 |

**Table 6**

| | 0 min | 15 mins | 30 mins | 45 mins | 60 mins | 90 mins | 120 mins |
|---|---|---|---|---|---|---|---|
| Control group | 94.125 | 193.125 | 159.125 | 143.375 | 130.125 | 113.250 | 102.750 |
| Vegetable powder mixture | 96.571 | 205.571 | 180.857 | 155.429 | 138.286 | 117.429 | 107.714 |
| Diabetic group | 544.000 | 601.000 | 590.400 | 585.200 | 577.900 | 573.700 | 573.400 |
| -control group | | | | | | | |
| Diabetic group -0.6 Mix | 441.714 | 538.000 | 525.571 | 512.286 | 495.286 | 479.286 | 476.571 |
| Diabetic group -1.2 Mix | 412.875 | 524.375 | 508.625 | 501.500 | 493.000 | 483.750 | 468.000 |
| Diabetic group -2.4 Mix | 368.600 | 507.600 | 498.100 | 485.100 | 470.000 | 452.900 | 419.300 |

**Table 7**

| | 0 min | 15 mins | 30 mins | 45 mins | 60 mins | 90 mins | 120 mins |
|---|---|---|---|---|---|---|---|
| Control group | 94.12 5 | 193.125 | 159.125 | 143.375 | 130.125 | 113.250 | 102.750 |
| Plus Vegetable powder mixture | 94.57 1 | 174.286 | 153.429 | 141.857 | 132.143 | 108.286 | 106.857 |
| Diabetic group - control group | 544.0 00 | 601.000 | 590.400 | 585.200 | 577.900 | 573.700 | 573.400 |
| Diabetic group - 0.6+Mix | 398.8 57 | 493.429 | 490.143 | 481.714 | 473.714 | 459.571 | 449.571 |
| Diabetic | 398.1 | 491.500 | 487.750 | 471.875 | 466.125 | 451.000 | 440.500 |
| group - 1.2+Mix | 25 | | | | | | |
| Diabetic group - 2.4+Mix | 347.7 78 | 487.000 | 477.444 | 464.000 | 448.333 | 412.000 | 395.111 |

As illustrated in FIG. 14, an experimental rat induced with diabetes showed impaired glucose tolerance, whereas the groups administrated with the D&D, the vegetable powder mixture, and the D&D + the vegetable powder mixture having high concentration showed improved glucose tolerance (see table 5 to table 7).

**Table 8**

| | 0 min | 15 mins | 30 mins | 45 mins | 60 mins | 90 mins | 120 mins |
|---|---|---|---|---|---|---|---|
| Control group | 111.375 | 105.250 | 100.500 | 95.125 | 88.250 | 91.375 | 90.375 |
| D&D | 116.500 | 110.625 | 96.125 | 95.125 | 92.000 | 93.125 | 94.125 |
| Diabetic group - control group | 558.500 | 556.100 | 544.700 | 540.900 | 539.700 | 537.000 | 530.700 |
| Diabetic group - 0.6D&D | 557.429 | 550.286 | 533.429 | 526.143 | 516.286 | 506.714 | 498.429 |
| Diabetic group - 1.2D&D | 502.857 | 497.714 | 485.143 | 478.714 | 464.286 | 453.286 | 459.333 |
| Diabetic group - 2.4D&D | 421.900 | 417.100 | 412.200 | 398.000 | 394.500 | 382.700 | 371.900 |

**Table 9**

| | 0 min | 15 mins | 30 mins | 45 mins | 60 mins | 90 mins | 120 mins |
|---|---|---|---|---|---|---|---|
| Control group | 111.375 | 105.250 | 100.500 | 95.125 | 88.250 | 91.375 | 90.375 |
| Vegetable powder mixture | 119.571 | 110.000 | 96.429 | 94.571 | 93.000 | 93.143 | 93.429 |
| Diabetic group - control group | 558.500 | 556.100 | 544.700 | 540.900 | 539.700 | 537.000 | 530.700 |
| Diabetic group - 0.6Mix | 508.286 | 505.857 | 492.429 | 480.286 | 469.429 | 452.714 | 445.857 |
| Diabetic group - 1.2Mix | 490.000 | 480.125 | 470.875 | 454.750 | 436.750 | 421.000 | 415.500 |
| Diabetic group - 2.4Mix | 412.000 | 394.000 | 380.500 | 371.800 | 366.100 | 354.100 | 350.000 |

**Table 10**

| | 0 min | 15 mins | 30 mins | 45 mins | 60 mins | 90 mins | 120 mins |
|---|---|---|---|---|---|---|---|
| Control group | 111.375 | 105.250 | 100.500 | 95.125 | 88.250 | 91.375 | 90.375 |
| D&D+ Vegetable powder mixture | 118.000 | 110.714 | 96.429 | 93.286 | 91.143 | 91.429 | 91.143 |
| Diabetic group - control group | 558.500 | 556.100 | 544.700 | 540.900 | 539.700 | 537.000 | 530.700 |
| Diabetic group - 0.6+Mix | 499.857 | 495.000 | 486.571 | 480.714 | 461.857 | 446.000 | 434.286 |
| Diabetic group - 1.2+Mix | 486.375 | 475.500 | 469.375 | 457.375 | 425.500 | 417.375 | 405.500 |
| Diabetic group - | 405.778 | 385.889 | 377.444 | 366.889 | 348.222 | 337.111 | 323.111 |
| 2.4+Mix | | | | | | | |

In addition, as illustrated in FIG. 15, an experimental rat induced with diabetes showed insulin resistance when administrated with insulin, whereas the groups administrated with the D&D, the vegetable powder mixture, and the D&D + the vegetable powder mixture having high concentration showed improved insulin resistance (see table 8 to table 10).

The determination results of experimental examples 1 to 7 using embodiments 1 to 3 are as follows.

Regarding the change in weight, the experimental rat induced with diabetes showed the reduction of the weight as compared with the normal group, and the groups administrated with the D&D, the vegetable powder mixture, and the D&D + the vegetable powder mixture having high concentration showed the increase of the weight as compared with the experimental rat induced with diabetes.

Meanwhile, a food intake per day on weekdays was increased in the experimental rat induced with diabetes as compared with the normal group and was decreased in the groups administrated with the D&D, the vegetable powder mixture, and the D&D + the vegetable powder mixture having high concentration. A water intake did not show the significance in the groups administrated with the D&D, the vegetable powder mixture, and the D&D + the vegetable powder mixture having high concentration.

Fasting blood glucose was significantly increased in the experimental rat induced with diabetes as compared with the normal group, but the groups administrated with the D&D, the vegetable powder mixture, and the D&D + the vegetable powder mixture having high concentration was significantly decreased.

The experimental rat induced with diabetes showed impaired glucose tolerance, whereas the groups administrated with the D&D, the vegetable powder mixture, and the D&D + the vegetable powder mixture having high concentration showed improved glucose tolerance.

In addition, an experimental rat induced with diabetes showed insulin resistance, whereas the groups administrated with the D&D, the vegetable powder mixture, and the D&D + the vegetable powder mixture having high concentration showed improved insulin resistance.

The concentration of C-peptide in blood was decreased in the experimental rat induced with diabetes, but was significantly increased in the groups administrated with the D&D, the vegetable powder mixture, and the D&D + the vegetable powder mixture having high concentration.

A beta cell in the pancreas was markedly damaged in the experimental rat induced with diabetes, but the area of Islets of the histopathology pancreas was increased in the groups administrated with the D&D, the vegetable powder mixture, and the D&D + the vegetable powder mixture having high concentration. In addition, according to the analysis result of insulin present in a beta cell of a pancreas organ by immunohistochemistry, the insulin expression of Islets was decreased in the rat induced with diabetes, but clearly increased in the groups administrated with the D&D, the vegetable powder mixture, and the D&D + the vegetable powder mixture having high concentration.

According to the embodiment and the experimental example, it is determined that the D&D, the vegetable powder mixture, and the D&D + the vegetable powder mixture having high concentration are importantly involved in regenerating or recovering the beta cell of the pancreas, which is an important result for finding out the mechanism of action of the effect of lowering blood glucose.

Meanwhile, B-cell lymphoma 2 (Bcl-2) is known as an antiapoptotic protein to promote cell survival, and a Bcl-2-associated X protein (Bax) is known as a proapototic protein involved in cell death resulting from DNA damage, and ischemia-reperfusion injury. Although the Bax protein presents in cytoplasm, if intracellular stress, such as DNA injury, is caused, the Bax protein migrates to a mitochondria outer membrane and liberates cytochrome-C. Accordingly, the activation of a caspase (cleaved caspase-3) is caused, so cell death is induced. Bcl-2, which is a protein to promote cell survival, is known to inhibit a Bax protein action.

According to a result of the molecular biology test of the present invention, when the D&D was individually administered to the rate induced with diabetes, the concentration-dependent expression increase in B-cell lymphoma 2 (Bcl-2), the decrease in the expression of the Bcl-2-associated X protein (Bax) protein, and the decrease in the expression of a cleaved caspase-3 protein are results to show an apoptosis inhibition effect of a beta cell in the pancreas by the D&D. When the diabetes are induced, the D&D protects the survival of the beta cell in the pancreas.

In addition, a mammalian target of rapamycin (mTOR) is known as a serine/threonine kinase that regulates cell growth, senescence, and cell metabolism. Particularly, 4EBP1 and p70S6K regulate the protein synthesis process and the representative substrate thereof has been known as an AKT. According to the result of the present research, when the D&D is individually administered to a rat induced with diabetes, the expression of a p-AKT and the expression of insulin were increased depending on the concentration of the D&D, and the expression of p-mTOR, p-p70S6K, and p4EBP1 was increased. Accordingly, this result may show that the individual administration of the D&D inhibits oxidative stress and improves a cell of the pancreas Islet damaged due to diabetes through the signal transfer mechanism of AKT/mTOR, thereby improving diabetic disease.

As described above, although the present invention has been described with reference to specific matters such as detailed elements, limited embodiments, and accompanying drawings, those skilled in the art can apparently understand that they are provided only for the illustrative purpose of the present invention.

Accordingly, the technical scope of the disclosure is not limited to the detailed description of the specification, but defined by the claims.

## Claims

1. A composition for use in treating Type 1 diabetic disease, the composition comprising a vegetable powder mixture prepared by mixing buckwheat powders, onion powders, and cacao nibs powders, and/or cricket (Gryllus bimaculatus) powders.

2. The composition for use according to claim 1, wherein the vegetable powder mixture is preferably prepared by mixing 30-50% of buckwheat powders, 25-40% of onion powders, and 10-25% of cacao nibs powders.

3. The composition for use according to claim 2, wherein the vegetable powder mixture and the Gryllus bimaculatus powders are mixed at a weight ratio of 1:1.

## Patentansprüche

1. Zusammensetzung zur Verwendung bei der Behandlung einer diabetischen Erkrankung vom Typ 1, wobei die Zusammensetzung eine pflanzliche Pulvermischung umfasst, hergestellt durch Mischen von Buchweizenpulvern, Zwiebelpulvern und Kakaonibspulvern und/oder Mittelmeer-Feldgrillen (Gryllus bimaculatus)-Pulvern.

2. Zusammensetzung zur Verwendung nach Anspruch 1, wobei die pflanzliche Pulvermischung vorzugsweise hergestellt ist durch Mischen von 30 - 50 % Buchweizenpulvern, 25 - 40 % Zwiebelpulvern, und 10 - 25 % Kakaonibspulvern.

3. Zusammensetzung zur Verwendung nach Anspruch 2, wobei die pflanzliche Pulvermischung und die Gryllus bimaculatus-Pulver in einem Gewichtsverhältnis von 1:1 gemischt sind.

## Revendications

1. Composition pour une utilisation dans le traitement d'une maladie diabétique de Type 1, la composition comprenant un mélange de poudres végétales préparé par mélange de poudres de sarrasin, de poudres d'oignon et de poudres d'éclats de fèves de cacao, et / ou de poudres de grillon (Gryllus bimaculatus).

2. Composition pour l'utilisation selon la revendication 1, dans laquelle le mélange de poudres végétales est de préférence préparé par mélange de 30 à 50 % de poudres de sarrasin, 25 à 40 % de poudres d'oignon et 10 à 25 % de poudres d'éclats de fèves de cacao.

3. Composition pour l'utilisation selon la revendication 2, dans laquelle le mélange de poudres végétales et les poudres de Gryllus bimaculatus sont mélangés à un rapport pondéral de 1 : 1.
